(19) Europäisches Patentamt
European Patent Office
Office européen des brevets

(11) EP 4 408 435 B1

(12) EUROPEAN PATENT SPECIFICATION

(45) Date of publication and mention of the grant of the patent:
**13.08.2025 Bulletin 2025/33**

(21) Application number: **22799860.6**

(22) Date of filing: **26.09.2022**

(51) International Patent Classification (IPC):
***A61K 31/7048*** (2006.01) ***A61M 11/00*** (2006.01)
***A61M 15/08*** (2006.01) ***A61M 15/02*** (2006.01)
***F24F 8/15*** (2021.01) ***A61P 31/20*** (2006.01)
***A61P 31/16*** (2006.01) ***A61P 31/14*** (2006.01)
***A61P 31/12*** (2006.01) ***A61P 31/04*** (2006.01)
***A61P 31/00*** (2006.01) ***A61M 15/00*** (2006.01)
***A61L 9/00*** (2006.01) ***A61K 31/35*** (2006.01)
***A61K 31/05*** (2006.01) ***A61K 31/015*** (2006.01)
***A61K 31/734*** (2006.01) ***A61K 31/732*** (2006.01)
***A61K 31/11*** (2006.01) ***A61L 9/01*** (2006.01)
***A61L 9/04*** (2006.01)

(52) Cooperative Patent Classification (CPC):
(C-Sets available)
**A61K 31/35; A61K 31/015; A61K 31/05; A61K 31/11; A61K 31/7048; A61K 31/732; A61K 31/734; A61M 11/005; A61M 15/02; A61M 15/08; A61P 31/00; A61P 31/04; A61P 31/12; A61P 31/14; A61P 31/16;** (Cont.)

(86) International application number:
**PCT/EP2022/076730**

(87) International publication number:
**WO 2023/052310 (06.04.2023 Gazette 2023/14)**

# (54) COMPOSITION COMPRISING HESPERIDIN, CITRAL AND SODIUM ALGINATE AS A FILM-FORMING SUBSTANCE FOR PROPHYLAXIS OF INFECTIONS

ZUSAMMENSETZUNG ENTHALTEND HESPERIDIN, CITRAL UND NATRIUMALGINAT ALS FILMBILDENDE SUBSTANZ ZUR INFEKTIONSPROPHYLAXE

COMPOSITION COMPRENANT DE L'HESPÉRIDINE, DU CITRAL ET DE L'ALGINATE DE SODIUM EN TANT QUE SUBSTANCE FILMOGÈNE POUR LA PROPHYLAXIE D'INFECTIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2021 EP 21200432**

(43) Date of publication of application:
**07.08.2024 Bulletin 2024/32**

(73) Proprietor: **MVS Pharma GmbH**
**70771 Leinfelden-Echterdingen (DE)**

(72) Inventors:
- **PROKSCH, Rainer**
**70771 Leinfelden Echterdingen (DE)**
- **GEORGAKIS, Michail**
**54453 Thessaloniki (GR)**
- **KÜNNEMANN, Eva**
**8400 Winterthur (CH)**

(74) Representative: **Flügge, Katharina**
**Meissner Bolte Patentanwälte Rechtsanwälte Partnerschaft mbB**
**Alter Wall 32**
**20457 Hamburg (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:

**AU-B2- 2013 204 006** **CN-A- 104 804 108**
**CN-A- 111 743 789** **CN-A- 113 142 535**
**JP-A- 2011 241 154** **JP-A- 2018 104 377**
**US-A- 5 302 399** **US-A1- 2022 072 031**

- **MENEGUZZO FRANCESCO ET AL: "Review of Evidence Available on Hesperidin-Rich Products as Potential Tools against COVID-19 and Hydrodynamic Cavitation-Based Extraction as a Method of Increasing Their Production", PROCESSES, vol. 8, no. 5, 9 April 2020 (2020-04-09), CH, pages 549, XP093014459, ISSN: 2227-9717, DOI: 10.3390/pr8050549**
- **ANDREA VERSARI ET AL: "Analysis of Some Italian Lemon Liquors (Limoncello)", JOURNAL OF AGRICULTURAL AND FOOD CHEMISTRY, vol. 51, no. 17, 1 August 2003 (2003-08-01), US, pages 4978 - 4983, XP093014455, ISSN: 0021-8561, DOI: 10.1021/jf030083d**
- **JAHAN ROWNAK ET AL: "Zingiber officinale : Ayurvedic Uses of the Plant and In Silico Binding Studies of Selected Phytochemicals With Mpro of SARS-CoV-2", NATURAL PRODUCT COMMUNICATIONS, vol. 16, no. 10, 1 October 2021 (2021-10-01), US, XP093014457, ISSN: 1934-578X, DOI: 10.1177/1934578X211031766**
- **PATNE TUSHAR ET AL: "INHALATION OF ESSENTIAL OILS: COULD BE ADJUVANT THERAPEUTIC STRATEGY FOR COVID-19", INTERNATIONAL JOURNAL OF PHARMACEUTICAL SCIENCES AND RESEARCH, 1 September 2020 (2020-09-01), XP055814710, Retrieved from the Internet <URL:https://ijpsr.com/wp-content/uploads/2020/08/3-Vol.-11-Issue-9-Sep-2020-IJPSR-RE-3819.pdf> [retrieved on 20210616], DOI: 10.13040/IJPSR.0975-8232.11(9).4095-03**
- **"ANTI-VIRAL COMPOSITION FOR ADMINISTRATION INTO NOSE, MOUTH OR THROAT ED - Darl Kuhn", IP.COM, IP.COM INC., WEST HENRIETTA, NY, US, 28 April 2021 (2021-04-28), XP013189901, ISSN: 1533-0001**

(52) Cooperative Patent Classification (CPC): (Cont.)
**A61P 31/20;** A61L 9/01; A61L 9/044; A61L 2209/16; A61M 11/042; A61M 2205/0205; A61M 2205/50; A61M 2205/588; A61M 2205/84; A61M 2209/10; A61M 2250/00; F24F 8/15

C-Sets
**A61K 31/11, A61K 2300/00;**
**A61K 31/7048, A61K 2300/00;**
**A61K 31/732, A61K 2300/00;**
**A61K 31/734, A61K 2300/00**

## Description

## BACKGROUND

The present invention relates to a composition comprising Hesperidin, Citral and sodium alginate as a film forming substance, this composition for use in the prophylaxis of an infection with an infectious particle, the infectious particle being a SARS-CoV-2 virus, a device comprising this composition, a delivery system comprising this composition and a system comprising this device and an air delivery system for delivering said composition.

Since a long time, diseases exist in human communities that are transmitted via air because of small particles/particulates, solid or liquid matter suspended in the air, which can pass through the air over time and distance. Diseases capable of airborne transmission include human and veterinary maladies. The relevant pathogens can be viruses or bacteria, where in general the whole particles are transmitted, or fungi, where mostly spores are spread.

Some virus examples causing diseases are the Influenza virus A and B (causing influenza infection), the Respiratory Syncytial Virus (RSV; causing RSV infection), the SARS-CoV-2 virus (causing Coronavirus disease 2019), Mycobacterium tuberculosis (causing Tuberculosis), paramyxoviruses as the Measles virus (causing Measles) and Varicella zoster virus (causing Chickenpox and/or Herpes zoster) as well as adenovirus of subtype B.

Examples of diseases which are caused by bacteria are e.g., scarlet fever and meningococcal infections.

Some fungus examples causing diseases are Aspergillus sp. (causing Aspergillosis) and Mucorales sp. (causing Mucormycosis).

The infectious particles may be spread through breathing, exhaling, talking, coughing, sneezing, or any activities which generate aerosol particles or droplets. Aerosols are aqueous, solid, or oily suspended matter, or tiny particles that because of their low weight may float in the air for a long time before they sediment. Droplets with a diameter of more than 5 μm sink rapidly in the air and are therefore only transmitted up to around one meter. However, droplets of saliva can also stick to objects or surfaces. They can also reach the interior of the body through one's hand if - after having contact to an infected object or surface - the hand touches the mucous membrane of mouth, nose, or eyes. Some pathogens can float in the air in droplets of very small size (<5 μm) for a long time and can thus be spread over great distances.

Coronavirus disease 2019 (COVID-19) is caused by severe acute respiratory syndrome coronavirus 2 (SARS-CoV-2). COVID-19 is a catastrophic disease that presently infects millions of people worldwide. To date, COVID-19 has spread in nearly all parts of the world and has even led to lockdowns in different areas. Since SARS-CoV-2 is propagated, people cannot meet and live as before. In several areas walking in town and shopping is only possible with a facial mask covering nose and mouth. Longer meetings with more people have been or are prohibited depending on the status of the pandemic situation. Concerts and other cultural events and activities where many people come together had been cancelled and new opportunities are and must be developed to make events possible in secure surroundings. Always there is a fear of infection. In the past, everyone could see the face including the mouth of their conversation partner, but this is mostly no longer possible.

Since the COVID-19 epidemic people use facial masks and/or shields to protect themselves against transmission of the SARS-CoV-2 virus. These facial masks and/or shields cover mouth and nose. Some facial masks, like FFP2 masks, have specificities on particle-filtering respiratory protection, many others have not, they can even be self-made.

In the prior art, mouth sprays are disclosed that claim general unspecific antiviral activity. However, even when using these sprays, contact between people is still not possible, as they are not specifically developed for the purpose of protection.

Accordingly, there is a need for products and/or methods which allow the inhibition of the airborne transmission of infectious agents or particles. Thus, the problem underlying the present invention is to provide products and/or methods which allow the inhibition of the airborne transmission of infectious agents or particles. Another problem underlying the present invention is to provide products and/or methods which can preferably combine two kinds of mechanisms against infectious agents or particles and to ensure that an antiviral mechanism can be provided for enough time. Another problem underlying the present invention is to provide products and/or methods which allow to overcome the pandemic situation in view of the SARS-CoV-2 virus, and which allow individuals to return to a more "normal" life as before the pandemic situation.

The invention is defined in the claims. Any subject-matter beyond the scope of the claims is not part of the invention and provided for reference or comparison only.

Any reference to a non-patentable method of treatment of the human or animal body by therapy using a certain compound or composition is to be understood as referring to said compound or composition for use in said treatment.

Meneguzzo et al. ("Review of Evidence Available on Hesperidin-Rich Products as Potential Tools against COVID-19 and Hydrodynamic Cavitation-Based Extraction as a Method of Increasing Their Production", PROCESSES, vol. 8, no. 5, 9 April 2020, page 549) disclose that hesperidin, a citrus flavonoid abundant in citrus peels, has an activity against SARS-CoV-2. The document further discloses a process for making a product called "IntegroPectin" from waste lemon peels which contains hesperidin and pectin, as well as the essential oils contained in the used citrus fruits.

**DESCRIPTION OF THE INVENTION**

This need is met by the present invention with a composition comprising Hesperidin, Citral, and a film forming substance, as defined in the claims, this composition for use in the prophylaxis of an infection with an infectious particles, wherein the infectious particle is a SARS-CoV-2 infection, a device comprising this composition, a delivery system comprising this composition and a system comprising this device and an air delivery system for delivering a composition including one or more protective, viral inhibiting substances, as defined in the claims, and related methods and uses.

In a first aspect, the problem underlying the present invention is solved by a composition comprising Hesperidin, Citral and a film forming substance, which is sodium alginate.

In a further preferred embodiment of the composition according to the present invention, the composition comprises at least one further excipient selected from the group comprising buffers, alcohols, preservatives, stabilizers, and matrix compounds.

In another preferred embodiment of the composition according to the present invention, the composition comprises at least one further substance selected from the group consisting of geranial, eucalyptol, limonene, thymol and carvacrol.

In a second aspect, the problem underlying the present invention is solved by a composition according to the present invention for use in the prophylaxis of an infection with an infectious particle, which is a SARS-CoV-2 virus.

According to the present invention for use in the prophylaxis of an infection with an infectious particle, the prophylaxis is the prophylaxis of an infection with the SARS-CoV-2 virus, preferably the prophylaxis of the transmission of SARS-CoV-2 viruses between individuals and/or the prophylaxis of an infection of one or more individuals via infected air and/or surface with the SARS-CoV-2 virus.

In a third aspect, the problem underlying the present invention is solved by a device, preferably a medical device, comprising a composition according to the present invention.

In a preferred embodiment of the device according to the present invention, the device is a mouth spray, a nasal spray, a bottle, a throat spray and/or includes nose drops, inhalants or a sprayable liquid composition.

In another preferred embodiment of the device according to the present invention, the device provides the composition by spraying, smoking, dispersing, nebulizing or atomizing, preferably with an amount of 0.01 to 10 μg, more preferably with an amount of 0.1 to 1.0 μg of Hesperidin per spray burst or inhalation hub.

In a fourth aspect, the problem underlying the present invention is solved by a delivery system including the composition according to the present invention.

In a preferred embodiment of the delivery system according to the present invention, the delivery system is an air purification system and/or an air conditioning system or an ultrasonic nebulizer, wherein preferably the air purification system and/or the air conditioning or the ultrasonic nebulizer system comprises at least one solid carrier, preferably a porous solid, a mineral, or a porous polymeric material, that comprises the composition and wherein the system optionally comprises a chemically gated system, preferably a ligand on a substrate, a permeable membrane, or a porous polymeric material and/or optionally comprises a programmable electronic dispenser.

In another preferred embodiment, the delivery system according to the present invention delivers the composition via air into a room or an architectural space.

In a further preferred embodiment, the delivery system according to the present invention comprises a chemically gated system, preferably a ligand on a substrate, a permeable membrane, or a porous polymeric material, and/or a programmable electronic dispenser.

In a fifth aspect, the problem underlying the present invention is solved by a system comprising a device according to the present invention and an air delivery system for delivering said composition.

Preferably, the air delivery system according to this aspect of the invention does not comprise a composition according of the present invention. Preferably, this composition can be delivered, preferably nebulized, through said air delivery system into a room or an architectural space. Preferably, the air delivery system comprises Hesperidin in a composition that is deliverable, preferably nebulizable, through said air delivery system into a room or an architectural space.

In another aspect of the present invention, the problem underlying the present invention is solved by a pharmaceutical preparation comprising the composition of the present invention.

In another aspect of the present invention, the problem underlying the present invention is solved by a method for the prophylaxis of the transmission of infectious SARS-CoV-2 particles between individuals comprising the step

(a) distributing a composition according to the present invention into an oral and / or nasal cavity of an individual or several individuals and / or into a, preferably closed, room or architectural space.

In another aspect of the present invention, the problem underlying the present invention is solved by a method for blocking a receptor of an infectious SARS-CoV-2 particle and / or for binding to a binding site of said SARS-CoV-2 particle

comprising the step

(a) distributing a composition according to the present invention into an oral and / or nasal cavity of an individual or several individuals and / or into a, preferably closed, room or architectural space.

In another aspect of the present invention, the problem underlying the present invention is solved by a use of a composition according to the present invention for the prophylaxis of an infection with an infectious SARS-CoV-2 particle from an infected individual to a non-infected or to an infected individual.

A composition according to the present invention preferably contains at least one substance which is Hesperidin with a binding energy between this substance and a binding site of an infectious SARS-CoV-2 particle not exceeding -6.0 kcal/mol, preferably not exceeding -6.5 kcal/mol, more preferably not exceeding -7.9 kcal/mol.

Furthermore, it is preferred that the composition according to the present invention is utilized, preferably according to a method according to the present invention, every 0.5, 1, 2, 3, 4, 5 or more hours.

In another embodiment, the composition according to the present invention is preferably an aqueous or non-aqueous solution or dispersion, or a phase stored in and delivered from a mineral, zeolite, or another porous sorbent with or without a permeable membrane.

In a preferred embodiment, the composition according to the present invention has a pH in the range from 3 to 9, preferably from 4 to 8, more preferably from 4.5 to 7.5, measured with MQuant. pH values of the present invention were measured with MQuant, (Supelco1.09535.001) pH indicator stripes, not bleeding, Universal indicator pH 0-14.

In a preferred embodiment, the composition according to the present invention comprises the film forming substance, preferably sodium alginate, within a concentration range of 0.2 to 5%, preferably of 1 to 3%, more preferably of 0.5 to 2%, in each case based on the total weight of the composition.

In a preferred embodiment, the composition according to the present invention comprises at least one fragrance and / or flavoring substance in a concentration of at least 0.001 % by weight, preferably of at least 0.01 % by weight, more preferably of at least 0.1% by weight, in each case based on the total weight of the composition.

In a preferred embodiment the composition according to the present invention has a viscosity in the range of 5-100 mPa·s, preferably in the range of 10 to 70 mPa·s, more preferably in the range of 20 to 50 mPa·s.

In a preferred embodiment, the pharmaceutical preparation according to the present invention comprising the composition according to the present invention is a spray, a lozenge, a pastille, a chewing gum, a lollipop or a liquid formulation, preferably a spray, most preferably a mouth spray, a nasal spray or a throat spray. In a more preferred embodiment, the pharmaceutical preparation according to the present invention is a mouth/nose spray. A mouth/nose spray according to the present invention can be used either in the mouth or in the nose, preferably in both, the mouth and the nose. It is further preferred, that the mouth/nose spray is applied to both, the mouth and the nose, within a short time period, preferably within 1 minute, preferably within 30 seconds, more preferably within 10 seconds.

The prophylaxis according to the present invention is the prophylaxis of an infection with the SARS-CoV-2 virus, and/or the prophylaxis of the transmission of SARS-CoV-2 viruses between individuals and/or the prophylaxis of an infection of one or more individuals via infected air and/or surface with the SARS-CoV-2 virus.

According to the present invention, it is further preferred that prophylaxis comprises the inhibition of the transmission of SARS-CoV-2 viruses via infected air or surface.

In a preferred embodiment of the present invention, the prevention of the transmission of SARS-CoV-2 viruses between two or more individuals or the prevention of the transmission of SARS-CoV-2 viruses via infected air or surface lasts for at least 0.5, 1, 2, 3, 4, 5 or more hours after the utilization of a composition, device, system, or method according to the present invention.

In a preferred embodiment of the present invention, the prophylaxis of the transmission takes place by blocking a receptor of a SARS-CoV-2 virus particle with the substance and / or by binding of the substance to a binding side of said virus. If e.g., the virus-receptor interaction is blocked, the virus cannot enter a cell to replicate, at least for a certain time period. On a SARS-CoV-2 virus there are about 100 spike glycoproteins that are binding to the receptor ACE2 (angiotensin converting enzyme) that is expressed on the epithelial cells of the airways. By binding or docking to the spike glycoproteins those cannot bind anymore to the ACE2 receptors on the epithelial cells of the airways. Infection and/or virus reproduction might be inhibited, accordingly. Additionally, it was measured that on average, there are on average 6.8 * $10^5$ viral RNA copies, and maximal 7.1 * $10^8$ viral RNA copies, what corresponds to the number of viruses per throat swab of infected, ill persons. The average viral load in sputum was measured to be $7.00 \times 10^6$ copies per ml, with a maximum of $2.35 \times 10^9$ copies per ml.

If e.g., the virus-receptor interaction is blocked, the virus is inactive as it cannot enter a cell to replicate, at least for a certain period of time. If the binding site of an infectious particle is blocked or occupied by a substance or composition, the particle also becomes inactive, at least for a certain period of time.

A composition according to the present invention comprises Hesperidin. The chemical formula of the flavonoid Hesperidin is:

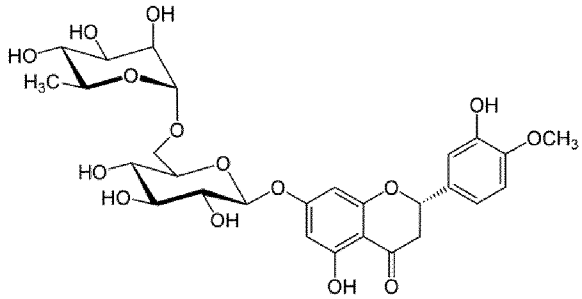

Hesperidin is a common flavone glycoside found in citrus fruits such as lemons and sweet oranges. As a glycoside from the flavanone hesperetin and the disaccharide rutinose, Hesperidin belongs to the group of flavonoids, specifically to the bioflavonoids.

Hesperidin is one of the active substances that bind to SARS-CoV-2 with very high affinity to the spike proteins. This has been measured by chemical docking experiments and a binding energy of -8.5 kcal/mol was found. This interaction leads to a steric hindrance of the binding of the virus to the receptor ACE2 (Basu et al.; Molecular docking study of potential phytochemicals and their effects on the complex of SARS-CoV2 spike protein and human ACE2. Sci Rep. 2020 Oct 19;10(1):17699. doi: 10.1038/s41598-020-74715-4. PMID: 33077836; PMCID: PMC7573581.). Consequently, infection of the cells is blocked. The Hesperidin molecules are very small compared to the SARS-CoV-2 virus and even to the spike proteins. However, there are six binding sites on each spike protein complex. On each virus around 100 spikes are found that may be covered upon contact with a composition according to the present invention.

Hesperidin has been used as an herbal medicine for a long time and it is often sold as a dietary supplement. It has several pharmacological activities such as anti-atherogenic, antihyperlipidemic, anti-diabetic, venotonic, cardioprotective, anti-inflammatory, and antihypertensive actions.

Concerning SARS-CoV-2, Hesperidin has three effects (Haggag et al.; Is hesperidin essential for prophylaxis and treatment of COVID-19 Infection? Medical Hypotheses. 2020 Nov;144:109957. DOI: 10.1016/j.mehy.2020.109957. PMID: 32531538; PMCID: PMC7274964.): The first effect is the inhibition of the attachment of SARS-CoV-2 to the ACE-2 receptor. This is essentially used through the composition according to the present invention and can occur at very low concentrations of Hesperidin as the binding affinity is very strong. The second effect is the inhibition of the production of inflammatory cytokines and the third one activates the cell immunity against infection.

A composition according to the present invention comprises Citral. The chemical formula of Citral is:

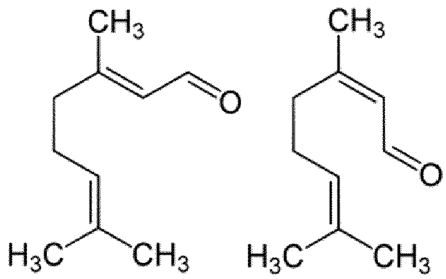

Citral is the mixture of the cis-trans isomers geranial (Citral A) and neral (Citral B). It is found in many essential oils such as citrus, lemongrass oil, basil and others. Citral has an intense fresh lemon scent and is therefore used as a fragrance and aroma substance.

In a composition according to the present invention Citral has two functions, first an ancillary inhibitory function on virus binding to human cells and second a function for the taste.

Citral is besides Hesperidin the second active substance in a composition according to the present invention that bind to the viruses (SARS-CoV-2) with very high affinity to the spike proteins. It has been measured in chemical docking experiments that preferably Geranial binds with very high affinity to spike proteins of SARS-CoV-2. A binding energy of -7.7 kcal/mol was found. This interaction leads to a steric hindrance of the binding of the virus to the receptor ACE2. Consequently, infection of the cells is blocked.

Upon use of the composition according to the present invention it is sprayed into the oral and nasal cavities. The blocking of the virus can occur for virus particles that are in the air and for such that are on the surface of the mucous membranes.

The Citral molecules are also very small compared to the virus and even to the spike proteins.

Through its second function Citral is preferably responsible for a light, fresh lemon aroma of a preferred

composition according to the present invention.

A composition according to the present invention comprises sodium alginate as a film forming substance.

The chemical formula of alginate is (shown are two sugar units of a long chain that are repeating):

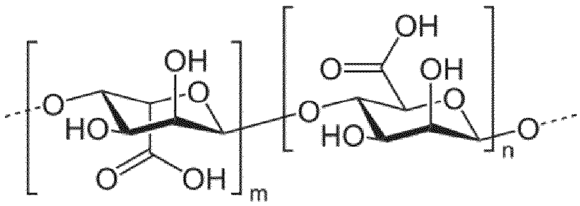

Alginate consists mainly of the sodium salt of alginic acid. Alginic acid is a mixture of polyuronic acids with alternating proportions of D-mannuronic acid and L-guluronic acid and is obtained from brown algae (Phaeophyceae). Sodium alginate is a white to pale powder that is slowly soluble in water to form a viscous colloidal solution. Calcium affects the microstructure of alginate. In solutions with a low calcium content alginate leads to an increase in viscosity and gellation.

It has surprisingly been found that a composition according to the present invention comprising sodium alginate as a film forming substance has a better effect, e.g in view of the prophylaxis of an infection with an infectious particle like SARS-CoV-2, than a composition only comprising one of the essential ingredients of the composition according to the present invention. Not to be bound to any theory, it could be that when entering the respiratory system, microdroplets including the film forming substance, preferably sodium alginate, will attach onto the mucous membranes of the mouth and the nose. In this way a protective film is formed on the mucous membranes of the mouth and nose. Preferably, the protective film formed onto the mucous membranes of the mouth and nose, after applying a composition according to the present invention onto the mucous membranes of the mouth and the nose, will stay there at least for 5 minutes, preferably at least for 8 minutes, more preferably at least for 12 minutes, most preferably at least for 15 minutes.

In view of sodium alginate, it has further surprisingly been observed that in solutions with a low calcium content sodium alginate leads to an increase in viscosity. As calcium is part of the saliva in the mouth in a concentration of 1.2-2.8 mmol/L and of the nasal mucus, when using a composition according to the present invention comprising sodium alginate, a thin antiviral mucoprotective film of calcium alginate is formed on the nasal and the oral mucosa, that forms a barrier for viruses.

In a preferred embodiment, the composition according to the present invention further comprises at least one additional substance selected from the group consisting of Naringin, Caflanone, Glycoside diosmin, Hesperetin, Myricetin, Linebacker, Equivir, 2-hydroxy - propyl- β- cyclodextrin, 2- hydroxy- propyl - γ- cyclodextrin, Randomly methylated β-cyclodextrin, Sulfobutyl ether β - cyclodextrin, Caffeic acid, Propolis, Thymol, Limonene, Myrcene, Carvacrol, Geranial, Eugenol, β- caryophellene, eucalyptol, p- cymene, linalool, 2,5,5 - Trimethyl- 3,6- heptadien, 2- ol, Glycyrrhizin, Resveratrol, Reserpine, Aescin, Cepharanthine, Theavlavin, Myricetin, Scutellarein, Chalcones I-IX, Emodin, Celastrol, Apigenin, Tomentin A, Anethol, Geraniol, Pulegone, Lycorine, Homoharrintonine, Silvestrol, Ouabain, Tylophorine, Aloe vera oil Tea tree oil, Persica components, (E,E) - α - farnesene, (E,E) - farnesol, (E) - Nerolidol, α- bulnesene, Eremanthin, β- sesquiphellandrene, (Z) - spiroether, (E) - cinnamyl acetate, (E) - β - farnecene, Geramyl formate, Cinnamaldehyde, Menthol, Carvacrol, Tomentin B, Hesperetin, Saikosaponin B2, Jubanine H, Iguesterin, APA, Belutonic Acid, Betulinic Acid, Curcumin, Indigo, Psoralidin, Tannic acid, Silvestrol, Gllycyrrhizin, Saikosaponin A, Tetra-O-galloyl-β-D-glucose, Luteolin, Sinigrin, β-sitosterol, amentoflavone, quercetin, isobavachalcone, tometin A, tometin B, tometin E, 3'-O-methyldiplacol, isoliquiritigenin, quercetin, kaempferol, kazinol F, broussochacone B, papyriflavonol A, terrestrimine, blancoxanthone, pyranojacareubin, tylophorine, 7-methosycryptopeurine, jubanine G, jubanine H, nummularine B, tingenone, iguesterin, pristimererin, dihydrotanshinone I, cryptotanshinone, tanshinone IIA, schimperinone, xanthoangelol, hirsutenone, rubranoside, curcumin, Santolina triene, α-pinene, camphene, viridiflorol, terpin-4-ol, 1,8-cineole, methylchavicol, camphor, myrcene, 1,8-cineole, β-caryophyllene, linalool, gerenyl acetate, β-himachalene, α-humulene, γ-himachalene, γ-terpin-7-al, γ-terpinene, β-pinene, cuminaldehyde, δ-cadinene, germacrene D, α-copaene, germacrene B, β-bisabolene, limonene, n-menthal, 8-dien-10-al, eugenol, carvacrol, cinnamaldehyde, viridiflorol, p-cymene, (Z)-caryophyllene, methylchavicol, trans-anthole, α-terpinyl acetate, β-phellandrene, Trans nerolidol, cis-dihydrocarvacol, eucalyptol, thymol, longifolene, thymoquinone, thymohydroquinone, α-thujene, p-cymene, borneol, camphene, camphor, gereniol, bornyl acetate, α-terpinolene, benzoylacetate, α-thujone, eugenylacetate, terpineol, β-thujone, terpine-4-ol, trans-nerolidol, β-eudesmol, Terpineol, α-terpineol, m-cymene, myrtenol, camphor, 1,8-cineole, carvacrol, α-bisabolol, hexadecanoic acid, α-pinene, β-pinene, β-phellandrene, δ-elemene, farnesene, α-curcumene, selina-4,7(11)-diene, β-bisabolene, piperitone, piperitenone, dillapiole, γ-terpinene, sabinene, thymol-methylether, sardinia:β-bisbolene, myacene, limonene, elemicin, aneocallitropsene, isocaryophyllen-14-al, 14-hydroxy-β-caryophyllen, caryophyllene oxide, E-β-caryophyllene, thymol, p-cymene, γ-terpinene, terpinen-4-ol, linalool, geraniol, trans-anethole, anethol, fenchone,

sabinone, terpinolene, safrote, trans-cinnamaldehyde, eugenol isomer, camphor and nigellone. Preferably, the at least one further substance is selected from the group consisting of geranial, eucalyptol, limonene, thymol and carvacrol. More preferably, the at least one further substance has a binding energy between this substance and a binding site of an infectious particle, preferably a virus, bacterium or fungus, not exceeding -6.0 kcal/mol, preferably not exceeding -6.5 kcal/mol, more preferably not exceeding -7.9 kcal/mol.

According to the present invention, a composition may include an essential oil, which could be hydrophobic. In this case, it is sometimes preferred that a carrier for the essential oil is comprised in the composition. Such a carrier could have the effect, that the interactions between the hydrophobic essential oil and the infectious particles which are carried in water droplets (as e.g. the SARS-CoV-2 virus particles) would not be prohibited (or at least become more possible) in air. Another preferred possibility according to the present invention would be that the essential oil of a composition according to the present invention is dispersed in water droplets, requiring a carrier, a surfactant or a supersonication.

According to the present invention, a method for the prophylaxis of the transmission of infectious SARS-CoV-2 virus particles between individuals comprising the step

(a) distributing a composition according to the present invention into an oral and / or nasal cavity of an individual or several individuals and / or into a, preferably closed, room or architectural space is presented.

In a preferred embodiment, the method according to the present invention comprises step (a-1) before step (a) takes place, wherein step (a-1) is

(a-1) distributing a composition according to the present invention into the, preferably closed, room or architectural space.

In a preferred embodiment, the method according to the present invention comprises additional step (b) after step (a), wherein step (b) is

(b) distributing a composition according to the present invention into the, preferably closed, room or architectural space as long as an individual or individuals is or are present.

According to the present invention the distribution step in a method according to the present invention is provided by spraying, smoking, dispersing, nebulizing or atomizing the composition, preferably with an amount of 0.01 to 10 μg, more preferably with an amount of 0.1 to 1.0 μg, of Hesperidin per spray burst or inhalation hub. It is further preferred that the amount of one spray burst, or inhalation hub is a drop/hub of about 50-100 μg of the composition. Preferably, the concentration of Hesperidin in the composition according to the present invention ranges between 0.5 and 100 μM, preferably between 1 and 50 μM, more preferably between 5 and 10 μM.

According to the present invention, a method is preferred, wherein the distribution in step a) takes place into the oral and / or nasal cavity, preferably of two or more individuals who are in spatial proximity to one another. However, individuals can get infected also without being in spatial proximity if the SARS-CoV-2 particle is able to stay airborne (suspended) in closed spaces.

Thus, it is possible to become infected even without being in close proximity to another person. However, the prophylaxis of the transmission of infectious particles according to the present invention works in this case as well. The method itself acts like a mask when it remains in the nasal and mouth cavities and covers also the mucous membranes.

In a preferred embodiment, the method according to the present invention is used, wherein one individual or at least one of several individuals has already been vaccinated against the infectious SARS-CoV-2 virus.

According to the present invention, the one individual or the individuals are, independently of one another, a human or an animal or a plurality of humans or a plurality of animals, preferably one or more humans.

According to the present invention, the room or architectural space is preferably an office or an office building, a school room, a hotel, an exhibition hall, a sports hall, an opera, a large event room, a shopping center or a concert hall, or a mode of transport, in particular a bus, a train, a taxi, a ship, an aircraft, or a plane. Preferably, the room or architectural space is closed. In another preferred embodiment, the room or architectural space is open.

According to the present invention, the, preferably closed, room or the architectural space is empty or 1, 2 or more individuals, preferably humans, are present.

In a preferred embodiment of the method according to the present invention, step (a) is executed every 0.5, 1, 2, 3, 4, 5 or more hours. It is further preferred that the prophylaxis of the transmission of infectious particles, the blocking of a receptor of an infectious particle or the binding to a binding site of an infectious particle, lasts for at least 0.5, 1, 2, 3, 4, 5 or more hours after executing step (a).

In a preferred embodiment, the method according to the present invention comprises an additional step, wherein a second binding or a crosslinking is achieved. Infectious particles, as e.g. prions, which consist mainly only of protein, could be inhibited by a complete masking and crosslinking by the ingredients of a composition according to the present invention on the surface of the infectious particles.

According to the present invention a use of the composition according to the present invention for recognition, preferably in the form of a recognition signal, alert or indicator, is presented. In a preferred use of a composition according to the present invention the composition has a specific smell. Through this specific smell of the composition according to

the present invention people when recognizing this specific smell can be sure that their direct surrounding is safe in view of an infection with the SARS-CoV-2 virus. According to the present invention, the smell of an inventive composition is preferably based on the smell of Citral, which combines the effects of inactivating an infectious particle, preferably a virus, and providing - by its smell - a form of a recognition signal, alert or indicator. In a preferred embodiment of the use according to the present invention, a specific smell provides the recognition signal, alert or indicator to individuals that they and/or their environment is or are safe in view of the SARS-CoV-2 virus. This use according to the present invention is preferably used when individuals are in closed rooms or in architectural space as an office or an office building, a school room, a hotel, an exhibition hall, a sports hall, an opera, a large event room, a shopping center or a concert hall, or a mode of transport, in particular a bus, a train, a taxi, a ship, an aircraft or a plane.

Thus, in a preferred embodiment, the use of a composition according to the present invention for recognition is wherein the recognition is a signal of security and / or protection. In other words: when a composition according to the present invention, preferably having a specific smell, is used in a closed room or in an architectural space as an office or an office building, a school room, a hotel, an exhibition hall, a sports hall, an opera, a large event room, a shopping center or a concert hall, or a mode of transport, in particular a bus, a train, a taxi, a ship, an aircraft or a plane, individuals recognizing this specific smell obtain the information, i.e. the signal, through this specific smell that this area is safe in view of the transmission of the SARS-CoV-2 virus.

According to the present invention an inventive delivery system comprising the composition according to the invention, preferably according to at least one of the preferred embodiments, in an air purification system and/or air conditioning system or an ultrasonic nebulizer is presented, wherein the air purification system and/or the air conditioning system or the ultrasonic nebulizer comprises at least one solid carrier, preferably a porous solid, a mineral, or a porous polymeric material, that comprises, preferably carries, the composition.

In a preferred embodiment, the delivery system according to the present invention comprises a chemically gated system, preferably a ligand on a substrate, a permeable membrane, or a porous polymeric material.

In a preferred embodiment, the delivery system according to the present invention comprises a programmable electronic dispenser.

In a preferred embodiment, the delivery system according to the present invention, is an air purification system and/or air conditioning system.

Preferably, the delivery system according to the present invention contains a means for delivering the inventive composition via air into an empty room, wherein the composition is delivered via air into an empty room and creates a distribution of the composition including Hesperidin in a sufficient concentration in the indoor air to obtain a "cleaned air". The distribution profile throughout a room depends heavily on the velocity of air coming out of the delivery system, e.g. the air conditioner, the mixing of the delivered air including the inventive composition with the, preferably "infected", air, i.e. air including infectious particles, and - highly important - the relative weights. Exhalation by an individual through normal breathing generally has lower velocities (this might be higher during coughing and sneezing or even working out intensely) and higher molecular weights (of the virus) to deal with, thus, if direct transmission of the infectious particles, as the SARS-CoV-2 virus, should be prohibited by a mouth and/or nose spray containing the composition, it has to be considered that by exhalation the infectious particles, as the SARS-CoV-2 virus, merely are emitted around the individuals and do not extensively distribute in the air (especially, the SARS-CoV-2 virus itself is too heavy to be dispersed and distributed over long distances. However, if people come into the area or surrounding where an infected individual has exhaled, they can become infected. Furthermore, it has to be considered that while the disclosed behavior is (mostly) true for non-moving air, most air is moving due to thermal gradients, and people moving in a space, thus there is always a spread of infectious particles, if present. In addition to that, the diffusion according to fick's law even under zero relative velocities has to be considered.

In a preferred embodiment, the delivery system according to the present invention is used in such a way that it allows efficient contact times and areas between the composition, which could be preferably hydrophobic, and water droplets so that the ingredients of the inventive composition are equally mixed or homogenized into/with the water droplets when distributed into the air. Preferably, the delivery system according to the present invention includes an ultrasonication mixer. This ultrasonication mixer may produce stable micro-dispersions of the inventive composition, preferably of therein included essential oils, in dispensers, which preferably may be used as sprays from air conditioning units. An advantage is that the micro-dispersions produced by the ultrasonication mixer preferably stay similar for a long time, preferably for at least a month.

According to the present invention, an alternative to an ultrasonication mixer is a high shear mixer, which may be used and provides the same advantages as an ultrasonication mixer.

In a preferred embodiment, the delivery system according to the present invention includes an electrosprayer that can produce an electro dynamic phenomenon (Taylor cone) directly from a container via the application of a suitable electrical current and voltage, and flow through an appropriate nozzle. The electrosprayer is extremely efficient in surface desinfection. In the case of the electrosprayer, the device is installed in the delivery system according to the present invention, preferably the air conditioning unit. This is in contrast to the embodiments including an ultrasonication mixer or a

high shear mixer where only the stable dispersion has to be placed in the air conditioning unit. The electrodynamic effect will preferably create mono-dispersions. This can preferably allow an optimization of the efficiency against the infectious particles, preferably the SARS-CoV-2 virus in the water droplets in the air.

In a preferred embodiment, the delivery system according to the present invention is construed in such a way that it allows a mixing process based on the use of small size, low molecular weight surfactants. A preferred mixture process with such surfactants will produce stable micro-dispersions of the inventive composition, preferably comprising at least one essential oil, in dispensers. Such dispersions could be used as sprays from air conditioning units and stay similar for a long time, preferably for at least a month. The appropriate surfactants will also be non-toxic, non-irritating and considered safe for use in food and air purification applications.

In a preferred embodiment, the delivery system according to the present invention is construed in such a way that it allows a mixing process based on the use of known safe polymeric carriers, e.g. with derivatives of β-cyclodextrin. A mixture process with such polymeric carriers will produce stable micro-dispersions of the inventive composition, preferably comprising at least one essential oil, in dispensers. Such dispersions could also be used as sprays from air conditioning units and stay similar for a long time, preferably for at least a month.

To maximize the contact time and areas of the dispersed inventive composition, preferably comprising at least one essential oil, to the water droplets a combination of at least two of the above mixing processes and/or mixing devices is preferred in a delivery system according to the present invention.

In a preferred embodiment, the delivery system according to the present invention is used comprising an inventive composition, preferably comprising at least one essential oil and/or other natural compounds disclosed herein. For such embodiment, a use of any of the dispersion and/or mixing techniques mentioned above is not obligatory, however, it is suggested to improve the contact efficiency of the given system. It is suggested, that using any of the above-mentioned techniques will also create a longer residence time of the inventive composition, preferably comprising at least one essential oil, in air due to two factors a) smaller sizes and reduction of aggregation, that will allow the lighter particles to stay airborne for longer times and thus it will provide a higher chance for interacting with an infectious particle, preferably a virus particle, and b) stabilization forces among the ingredients of the inventive composition due to supersonication, surfactants, carriers, etc. will allow for a lighter distribution in the indoor environment. For most cases the supersonication is preferred, and it consumes less energy.

According to the present invention, a further substance in a composition according to the present invention, can be an antibody, a fragment of an antibody, a nanobody, an artificial molecule or the like.

The present invention presenting the described products, methods and uses including variants thereof may be favourable in different scenarios. One specific scenario could be within a hospital or retirement home where many susceptible people are living, and viral diseases can occur. A mouth/nose spray according to the present invention can be used as protection against viral load additionally to facial masks. Another specific scenario could be the public transport system as e.g. airplanes where passengers from many different countries and regions come together. A mouth/nose spray according to the present invention can be used additionally to facial masks and/or to the ventilation of the composition according to the present invention or another composition including one or more protective, viral inhibiting substances throughout the plane. A further specific scenario could be the scenario of large events where thousands of people come together like in football stadiums. The composition according to the present invention or another composition including one or more protective, viral inhibiting substances, preferably at least Hesperidin, could be distributed via an air system, preferably according to a delivery system of the present invention, and/or all guests must pass a tunnel where the composition according to the present invention or another composition including one or more protective, viral inhibiting substances, preferably at least Hesperidin, is present in the air (through e.g. nebulization). According to this scenario, a mouth/nose spray according to the present invention could be used additionally and preferably regularly by the people, so that viral load is reduced, and prophylaxis is obtained.

The term "via infected air or surface" as used herein refers to that infectious particles can be transmitted from one individual to another via infected air, e.g. air having infectious particles airborne (suspended) in the air or via infected surfaces, e.g. by finger contact on a surface whereon infectious particles are attached.

The term "infectious particles" as used herein refers to particles of viruses, bacteria or fungi or viruses, bacteria or fungi - in each case - as a whole, which can cause diseases or illnesses in individuals, preferably humans.

The present invention is further illustrated by the following figures and examples from which further features, embodiments, aspects and advantages of the present invention may be taken.

## BRIEF DESCRIPTION OF THE FIGURES

**Fig. 1** shows a scheme for a method for the prophylaxis of the transmission of infectious particles via air according to the present invention.

In Fig. 1 transmission of infectious particles via air is inhibited by using a room/air spray according to the present invention when being in spatial proximity to one another.

Fig. 2 shows another scheme for a method for the prophylaxis of the transmission of infectious particles via air according to the present invention.
In Fig. 2 transmission of infectious particles via air is inhibited by using a mouth and/or nose spray according to the present invention when being in spatial proximity to one another.

Fig. 3 shows M structure (L- guluronic acid) as part of alginate in HyperChem.

Fig. 4 shows G structure (D- mannuronic acid) as part of alginate in HyperChem.

Fig. 5 shows Hesperidin in HyperChem

**Fig. 6** shows MMGG, M structure (L- guluronic acid) G structure (D- mannuronic acid) as part of alginate, in HyperChem.

**Fig. 7** shows MMMGGG, M structure (L- guluronic acid), G structure (D- mannuronic acid) that represents the alginate network in HyperChem.

**Fig. 8** shows Hesperidin in 6 MMMMMGGGGGG, M structure (L- guluronic acid), G structure (D- mannuronic acid) that represents the alginate network, in HyperChem.

**Fig. 9** shows the antiviral function of a composition according to the present invention schematically.

In Fig. 9 A viral particles with spike proteins at their surface bind to the ACE2 receptor on the surface of mucosal cells. Subsequently, they will enter the cells and cause infection.

Fig. 9 B shows the formed alginate network, when a composition according to the present invention is contacted with mucosa cells comprising $Ca^{2+}$. Inhibitory substances like Hesperidin and Citral stay in the network and can diffuse therein.

In Fig. 9 C it is shown that viral particles cannot pass the network or even get caught within it.

In Fig. 9 D it is shown that the alginate network might tear, get holes, and therefore not stay complete. However, the inhibitory substances like Hesperidin and Citral that bind with very high affinity to the spike proteins of viruses like SARS-CoV-2. This binding inhibits the binding of the spike proteins of the virus to the ACE2 receptor on cells. Subsequently, entering of the virus into the cells is blocked and therefore also infection of the cells.

## EXAMPLE

In this example the development of the invention has been done in four steps. First, molecular docking experiments were done to find the best antiviral binding substances that inhibit virus attachment to human cells. In the second step the best binding molecules were chosen and the binding affinity of these substances to the viral surface proteins were calculated. In a third step a composition containing the dosage of these antiviral substances was developed with an appropriate buffer. In a fourth step film forming substances to assure long-term presence of the antiviral substances were included and it was tested for the best appropriate film formation. Chemical simulations then confirmed the functionality.

Step 1 - Docking approach

A molecular docking approach was employed to calculate the binding energies of a group of essential oils on surface proteins of SARS-CoV-2 that utilizes exact, atomistic scale models of the ligands (essential oils) and the target molecules (SARS-CoV-2 proteins). It calculates their interactions based on hydrogen bonding, dispersion forces, London forces, hydrophobic interactions, and electrostatic interactions.
The first step was to create the essential oils models. Those models were created in HyperChem v8, HyperCube Inc., USA. All models were generated and optimized under Density Functional Theory, mainly to obtain the exact structures, including their electronic charge distribution. Twelve essential oils were created via this approach, with a

target to continuously enrich this collection. The model creation process included drawing, initial modelling, a geometry optimization and a molecular dynamics step. Geometry Optimizations lead to energetic minima, which in most cases are 'local' ones, whereas the 'global' minima are of the highest importance. For that reason, a Molecular Dynamics step was employed after each Geometry Optimization, to 'break free' of the energy trap and continue the search for a 'deeper' energetic minimum. This methodology was repeated up to twenty times for each essential oil molecular model generated.

The second step was the preparation of the SARS-CoV-2 proteins that are the binding targets of the essential oil molecules. Four proteins, namely (PDB ID): RdRp (6M71), Spike ectodomain (6VYB), PIPr (6W9C) and MPro (6Y2E) were employed. The crystallographic structures are offered free of charge in a number of databases, and the Protein Data Bank (RCSB.ORG) was used as source. A series of preparation modification were carried out in Chimera environment (CSF Chimera, developed by the Resource for Biocomputing, Visualization, and Informatics at the University of California, San Francisco, with support from NIH P41-GM103311) under the AMBER FF14SB force field:

- Chain A of the RdRp protein was used (6M71_A)
- N-acetyl glucosamine was removed from the spike protein (6VYB)
- Chain A of MPro was used (6Y2E_A)
- Missing protons were added where required
- Crystallographic water molecules were removed where required

The generated models were in mol2 format and were fed to AutoDockTools software (v4.2, The Scripps Research Institute, USA), which in turn generated pdbqt files for AutoDock Vina (O. Trott, A. J. Olson, AutoDock Vina: improving the speed and accuracy of docking with a new scoring function, efficient optimization and multithreading, Journal of Computational Chemistry 31 (2010) 455-461). The pdbqt format preserves the charges from the mol2 files.

The third step was to use AutoDock Vina to execute the molecular docking simulations of the 12 ligands vs the 4 proteins. The Lamarckian genetic algorithm 24 was adopted for the process, using 10-40 evaluations for run and 25 million iterations producing the results. The iterative local search method of AutoDock Vina was used for calculating the interaction potentials in the system. An initial low spacing was used, which was substituted with a much finer one after the initiation of the process, to produce higher accuracy results. Analysis of all hits and their binding energies was the last step of the process.

The essential oils that were studied, accompanied by the best binding energies to the four proteins are presented in **Table 1.** The multiplicity exhibits the number of binding pockets in the total of the four proteins.

**Table 1:** Molecular Docking results for the 12 essential oils vs RdRp, MPro, Spike and PIPr of SARS-CoV-2

| **Compound** | **Binding energy [kcal/mol]** | **Multiplicity [# of sites in total]** |
|---|---|---|
| Geranial | -7.7 | 6 |
| Eucalyptol | -8.3 | 6 |
| Caffeic acid | -6.1 | 3 |
| Myrcene | -6.0 | 3 |
| Limonene | -7.4 | 4 |
| Thymol | -7.3 | 4 |
| Hesperidin | -8.5 | 6 |
| Carvacrol | -5.7 | 2 |
| Linalool | -5.5 | 2 |
| B-caryophyllene | -6.1 | 3 |
| Hesperitin | -5.4 | 2 |
| Curcumin | -6.9 | 2 |

**Step 2** - **Calculation of Dissociation constant**

Calculation of dissociation constants from binding energies

For the binding of receptor and ligand molecules in solution, the molar Gibbs free energy, or the binding affinity is related to the dissociation constant via

$$\Delta G = RT \ln \frac{K_d}{c^{\ominus}},$$

| | |
|---|---|
| $\Delta G$: | molar Gibbs free energy |
| $R$: | ideal gas constant, R= 8.314 J $K^{-1}$ $mol^{-1}$ (=1.9872 * $10^{-3}$ kcal $K^{-1}$ $mol^{-1}$) |
| $T$: | temperature in K, for example at 20°C: T = 273 K + 20 K = 293 K |
| $K_d$: | dissociation constant |
| $c^e$: | standard reference concentration, $c^e$ = 1 mol/L |

Reforming the term to calculate $K_d$ from $\Delta G$ yields the following equation:

$$K_d = e^{\Delta G/RT} * c^{\ominus}$$

For example, if the binding energy is -7.7 kcal/mol, then:

$$K_d = e^{\frac{-7.7\ kcal*mol^{-1}}{1.9872\ *\ 10^{-3}\ kcal\ K^{-1}\ mol^{-1}\ *\ (293\ K)}} * 1\ mol/L\ = 1.8 * 10^{-6}\ M$$

The smaller the dissociation constant $K_d$, the higher the affinity.

The following equation allows the calculation of the percentage of occupancy of a protein with its ligand as a function of $K_d$ and the total concentrations of Protein P and Ligand L in solution from the chemical binding reaction:

$$P + L \underset{k_{off}}{\overset{k_{on}}{\rightleftarrows}} PL$$

$$\frac{[P]\cdot[L]}{[PL]} = \frac{k_{off}}{k_{on}} = K_d$$

If there is much more ligand than protein, the concentration of free ligand stays approximately constant during the reaction. Therefore, if in this case $K_d$ and the starting concentration of ligand [L] are known, one can calculate the percentage of protein that has a ligand bound.

For example: If the starting concentration of the ligand is 2.2*$10^{-4}$ M = 0.22 mM and as in the above example $K_d$= 2.2 * $10^{-7}$ M this yields the information that the starting concentration is 1000 x $K_d$ and that consequently 99.9% of proteins have a ligand bound.

From a known $K_d$ also a starting concentration can be chosen in this way, that it is for example 100 x $K_d$ and thereby it can be achieved that as a result 99% of proteins can be blocked with a ligand.

**Table 2:** Dissociation constant for 5 compounds

| **Compound** | Dissociation constant [M] | Concentration 100x $K_{diss}$ [M] | Solubility in water [M] |
|---|---|---|---|
| Geranial | 1.8*$10^{-6}$ | 1.8*$10^{-4}$ | 2.8*$10^{-3}$ |
| Eucalyptol | 6.4*$10^{-7}$ | 6.4*$10^{-5}$ | 21*$10^{-3}$ |
| Limonene | 3.0*$10^{-6}$ | 3.0*$10^{-4}$ | 1*$10^{-4}$ |
| Thymol | 3.6*$10^{-6}$ | 3.6*$10^{-4}$ | 6*$10^{-3}$ |
| Hesperidin | 4.5*$10^{-7}$ | 4.5*$10^{-5}$ | 1.3*$10^{-4}$ |

In Table 2 the concentration to block 99.9% of proteins with a ligand were calculated. By comparing this concentration to the solubility of the substances in water it can be concluded that even higher concentrations than 1000 x $K_d$ can be used in aqueous spray solution. The substances are not toxic in these concentrations.

However, it has additionally to be considered, that when the solution is sprayed into the nose and/or the oral cavity

it will be perhaps be diluted with saliva. Even if this dilution is 10 times (for example 100 µl solution is sprayed into a mouth with 1 ml saliva), the concentration of the substance will be > 100 x $K_d$ and the amount of blocked proteins would be >99%.

The following calculation shows that the concentration of the substances is much higher than the protein concentration on the viruses, where there is a maximum of $2.35 \times 10^9$ copies per ml. On each virus there are about 100 spike proteins, therefore there would be a concentration of about $2.35 \times 10^{11}$ spike proteins per ml.

The concentrations of binding substances would be around $1*10^{-3}$ M.

$1*10^{-3}$ M = $1 * 10^{-3}$ mol/l = $1 * 10^{-3}$ ($6.022 * 10^{23}$) molecules/l = $6.022 * 10^{20}$ molecules/l = $6.022 * 10^{17}$ molecules/ml.

Therefore, there will be around one hundred million times ($10^8$ x) the number of substance molecules compared to spike protein molecules and at least one million times ($10^6$ x) the number of substance molecules compared to viruses.

**Step 3: Development of the dosage form**

**Experimental materials - buffers**

For 1 L citric acid buffer, pH 4.5, 6.76 g sodium citrate dihydrate and 5.19 g citric acid were weighted and added to a 1 L beaker and filled with about 800 ml of sterile water. This mixture was stirred until the salts were dissolved. The pH was checked to be 4.5 and the volume was filled to 1 L.

For 1 L benzoate buffer, 1.22 g benzoic acid and 1.95 g sodium benzoate were weighted and added to a 1 L beaker and filled with about 800 ml of sterile $H_2O$. This mixture was stirred until the salts redissolved. The pH was checked to be 4.5 and the volume was filled to 1 L.

PBS (phosphate buffer saline), pH 7.0 was prepared according to known procedures. To 800 mL of distilled water in a suitable container and 8 g of sodium chloride, 0.2 g of potassium chloride, 1.44 g of sodium phosphate dibasic, and 0.245 g of potassium phosphate monobasic was added. The solution was stirred until all salts were dissolved. The pH was controlled and if necessary adjusted to 7.0. Then distilled water was added until the volume was adjusted 1 L.

**Selection of an appropriate buffer system**

In a first approach Hesperidin and Citral were dissolved in a) citric acid buffer and b) benzoate buffer to 5 µM Hesperidin and 2 mM Citral, where they are soluble and sufficient in concentration to block a high percentage of spike proteins of SARS-CoV-2. The concentration was reached by addition of 3.05 mg Hesperidin and 342 µl Citral to finally 1 Liter of the buffer solution. At a first glance acid pH that has an antiviral effect looked effective, as the virus-inhibiting activity of the inhibiting substances could be supported additionally. Therefore, solutions with citric acid buffer and benzoate buffer were made.

However, as the Hesperidin is a flavone glycoside, see formula I (chemical formula of the flavonoid Hesperidin), and the molecule contains hydroxide groups (OH-), these might change their protonation state at acid pH.

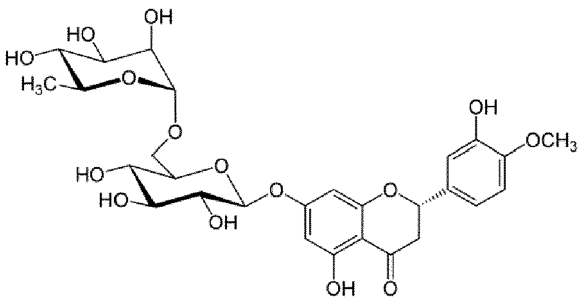

formula I

The ketone group (C=O) of Citral, see formula II (chemical formula of Citral, that is a mixture of the cis-trans isomers geranial (citral A) and neral (citral B)), might also protonated at acid pH.

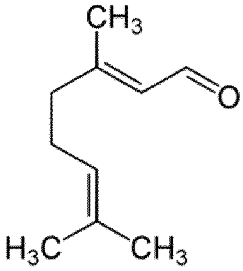 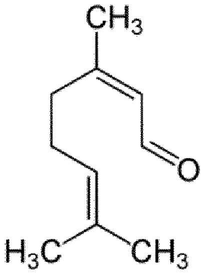

formula II

The chemical simulations for the binding of Hesperidin and Citral to the spike proteins of SARS-CoV-2 were made assuming a neutral pH. Therefore, the binding will occur at neutral pH and also then the blocking of the virus binding to the ACE2 receptor. For this reason, PBS (phosphate buffer saline), a buffer that stabilizes neutral pH and is easy to use in humans was chosen.

Thus, in a second approach Hesperidin and Citral were dissolved in PBS at pH 7 to 5 μM Hesperidin and 2 mM Citral. The concentration was reached by addition of 3.05 mg Hesperidin and 342 μl Citral to finally 1 Liter of the buffer solution. The composition containing 5 μM Hesperidin and 2 mM Citral in PBS at pH 7 was filled in spray bottles. It could easily be sprayed in mouth and nose. According to the present invention, both respiratory ways should be blocked to get an efficient inhibition of a viral attack. The solution has a fresh citric and a light salty taste. However, the effect was not lasting for long, more than a few minutes.

Therefore, an additional means was searched to present the inhibiting substances for a longer period. There is the possibility to include the inhibiting substances into a solid from where it is dissolved or excavated slowly in the mouth like from a lonzage or a chewing gum. However, this solution does only apply to the mouth and not to the nose. In addition to liquid and solid there is also the gaseous medium, where volatiles stay for certain periods.

A further possibility are gels that show characteristics of solids and liquids. There are mouth and nose sprays on the market that use film forming substances that have gel-like characteristics and also substances that are used to apply a medicine slowly via an oral film. To explore if this would be an efficient way to hold the inhibiting substances for a longer period in mouth and nose different film-forming substances were tested.

**Step 4: Testing of four film-forming substances**

The following four substances were tested for taste, film formation and spraying behavior: glycerol, hyaluronic acid, pectin and sodium alginate. Glycerol, hyaluronic acid and pectin are reference substances. Glycerol is a viscous, hyaluronic acid, pectin and sodium alginate are solid and were obtained as powder. They were used in viscous solution as described below. The substances glycerol, hyaluronic acid and pectin do not change their viscous state when being sprayed in the mouth and nose. Sodium alginate shows a change to a more viscous form once it is mixed with sputum or nasal mucus. Calcium is part of the saliva in the mouth in a concentration of 1.2-2.8 mmol/L and of the nasal mucus. Calcium affects the microstructure of alginate in solutions with a low calcium content and leads to an increase in viscosity.

**Dissolution of film forming substances**

120 ml of glycerol were diluted with PBS containing 5 μM Hesperidin, 2 mM Citral to 1 L to give a final concentration of 12% glycerol. 20 g of hyaluronic acid were mixed into 1 L of PBS containing 5 μM Hesperidin, 2 mM Citral to give a final concentration of 2% hyaluronic acid. 30 g of pectin were mixed into 1 L of PBS containing 5 μM Hesperidin, 2 mM Citral to give a final concentration of 3% pectin. It was necessary to stir the solution vigorously and add the pectin powder very slowly so that it got dissolved to a clear solution. Next 5, 10 or 20 g of sodium alginate were mixed into 1 L of PBS containing 5 μM Hesperidin, 2 mM Citral to give a final concentration of 0.5, 1 or 2% sodium alginate. It was necessary to stir the solution vigorously and add the sodium alginate powder very slowly so that it got dissolved to a clear solution.

Calcium alginate was made by adding dropwise a solution with 5 mM calcium carbonate to the dissolved sodium alginate. Immediately a gel was formed.

**Testing of taste**

The taste of glycerol, hyaluronic acid, pectin and sodium alginate at the above mentioned concentrations were tested in water, buffer and the composition with 5 μM Hesperidin and 2 mM Citral. Glycerol had a special sweet taste. Hyaluronic acid, pectin and alginate tasted neutral.

## Testing of spraying behavior

The solutions containing the film-forming substances were added to spray bottles and the spraying behavior was tested. The solution containing 12% glycerol was nebulized well upon spraying. The solution with 2% hyaluronic acid exited the spray bottle like a beam and was not well nebulized. The solution containing 3% pectin was nebulized well upon spraying. For sodium alginate samples with concentrations ranging from 0.5 to 2% were found to be nebulizing well, if the viscosity of the product is in a range of 20-50 mPa·s.

A spraying test of the film forming substances in water or without Hesperidin and Citral was made and there was no change in the spraying behavior.

## Film dissolution

The solutions with pectin and sodium alginate were sprayed on a spoon and this left in aqueous solution to see how fast the film gets dissolved. To mimic the situation in mouth and nose sodium alginate was sprayed on a spoon covered with 2 mM calcium solution. The film where the solution containing pectin was sprayed had disappeared from the spoon completely after 5 minutes. The film on the spoon where the solution containing the sodium alginate was sprayed, disappeared after 30-60 minutes.

**Table 3: Summary of the tests with film-forming substances**

| Substance | Concentration | Spraying behavior | Period till film dissolved | Taste |
|---|---|---|---|---|
| Glycerol | 12 % | Well nebulizing | Not known | Special sweet taste |
| Hyaluronic acid | 2 % | Like a beam - not nebulizing | Not known | neutral |
| Pectin | 3 % | Well nebulizing | 1-5 minutes | neutral |
| Sodium alginate | 0.5-2 % | nebulizing at concentration between 0.5-2 % depending on product viscosity | 30-60 minutes (when mixed with calcium) | neutral |

In **Table 3** a summary of the test with the film-forming substances is presented.

Sodium alginate showed good nebulizing behavior upon spraying and formed a long-lasting film when sprayed onto calcium-containing surface.

## Chemical simulations to confirm the behavior of the inhibiting substances in the film

To control if the binding behavior and the inhibition of viruses of the inhibiting substance Hesperidin would be still given when it is included in an alginate film, the following analysis was made in HyperChem with alginate, calcium and Hesperidin.

<u>Description of the analysis</u>

Hesperidin was prepared in HyperChem and used in combination with an increasing number of sodium alginate chains surrounding it, to investigate its ability to 'escape' (diffuse through) the cell / layer produced. The thicknesses that were of interest at this point were 20, 50, 100, and 200 μm, in agreement with expectations from data published in the medicinal/pharma publications. Thicker films would impose much longer diffusion times than our maximum sizes.

- As a first point, diffusion was indeed heavily dependent on the 'porous network' of the hydrogel. When water molecules were used to fill the cavities between the polymeric chains, pores in the 15-50A were most useful for transportation as expected.
- Diffusion for the same pore sizes is promoted by larger segments, and this is probably to the higher level of tortuosity and complexity in the case of smaller pore sizes (when fewer Ca cations have been used or mainly when MMGG, MMMGGG segments were used instead of MMMMMGGGGG).

- Hesperidin length and size is around one third of the respective properties of the MMMMMGGGGG segment. It is also not participating in electrostatic or chemical interactions (at least in the system built as described) and this allows for a much higher mobility that that of the rest of the polymeric segments.
- Overall, Hesperidin diffuses slowly through the network, and under the environments and assumptions employed here, it could only escape the film in reasonable times for very narrow films, in the 10-15 μm range.
- The kinetic diameter of Hesperidin was found (MM+, DFT can offer more accurate results in the 5.5-6A range)
- Experimental data also support this fact, especially for acidic-neutral pH values. It has been reported that under such conditions, no release of actives had been observed from such hydrogels. The opposite is true in harsher pH conditions (below its pKa, 3.38-3.65).

Altogether these chemical simulation experiments show that Hesperidin stays in an alginate film and can diffuse therein. Therefore, it could bind with high affinity to viruses and block the spike proteins as in aqueous solution measured before. The formation of the film assures the stay of Hesperidin on the mucous membranes of mouth and nose for periods of 30-60 minutes.

<u>Method of the chemical simulation analysis on HyperChem</u>

Description of the simulation work on HyperChem:

- The two components of alginate acid and Hesperidin were designed and optimized (see Figures 3 to 8).
- All initial models were built under MM+ due to time constraints.
- Larger segments of the alginate acid were built based on merging of existing ones, and subsequent re-optimization: MM, MMM, GG, GGG, MMGG, MMGGG, MMMGGG, MMMMMGGGGG.
- Geometry optimizations were run on all of them with a final RMS gradient of 0.001.
- Several X $Ca^{2+}$ - alginate systems were built, with X being from 1 to 4 per MMGG segment (using the 4 part body for simplicity; some decimal ratios have also been employed).
- The crosslinking of all Ca - alginate systems have been studied, and the distances of chains have been acquired.
- Some high accuracy DFT calculations were also performed, to identify localized electrostatic interactions. However, these would take significantly longer times, and are not completed in time to be included in this report.
- The acid forms were formed by removing the -OH hydrogen from the carboxyl group and assigning a formal charge of -1 in the remaining Oxygen group. In MM+ field this action preserves the -1 charge on the oxygen, and thus electrostatic interactions are stronger and more localized than we would observe in DFT or Ab Initio calculations, where the charge would diffuse over the polymeric chain at some (low) extend. In general, we can be very sure, that the longer the chains, the better the agreement among MM+ and DFT/ Ab Initio calculations.
- The calcium cations were also added as calcium with a formal of +2 in MM+ field.
- Models built (some) are shown in the figures, along with some interactive builds.
- layer thickness, size of chains, electrostatic interactions, and pore sizes were all taken into account.

**The antiviral function of a composition according to the present invention**

In Figure 9 the antiviral function of a composition according to the present invention is presented schematically. It is shown that viral particles with spike proteins at their surface bind to the ACE2 receptor on the surface of mucosal cells (Fig. 9 A). Subsequently, they will enter the cells and cause infection.

The composition according to the present invention containing sodium alginate, Hesperidin and Citral is applied to the oral and nasal mucosa, preferably by spraying.

Calcium alginate has been shown to be antiviral (ACS Appl. Bio Mater. 2021, 4, 8, 5897-5907). However, calcium alginate cannot be sprayed as its consistency is gel-like or solid. The reason for this is calcium ions have two positive loads, whereas sodium ions just have one positive load. Therefore, the calcium ions crosslink the alginate carbohydrate chains that have negative loads to a three-dimensional network.

According to the present invention the surprisingly found trick is used to spray sodium alginate and bring it together with calcium in a body fluid. Upon use of the composition containing sodium alginate and the inhibitory substances a gel-like film on the mucosa is formed. Alginate is formed of long carbohydrate chains. Once, they get in contact with calcium, that is present in sputum and nasal mucous, an ordered network is formed. Calcium ions have two positive loads, whereas sodium ions just have one positive load. Therefore, the calcium ions can crosslink the alginate carbohydrate chains that have negative loads to a three-dimensional network. Alternatively, calcium can be sprayed simultaneously from a separate container or pouch. The chemical simulations show the formed network and that the inhibitory substances will stay in this network and can diffuse therein (see also Fig. 9 B).

Fig. 9 C shows that viral particles cannot pass the network or even get caught within it.

The mouth and nose are not static and especially within the mouth many movements occur. Thereby, a network formed by alginate might tear, get holes, and therefore not stay complete (compare Fig. 9 D). For this reason, the applied composition according to the present invention has a second antiviral function. This depends on inhibitory substances like Hesperidin and Citral that bind with very high affinity to the spike proteins of viruses like SARS-CoV-2. This binding inhibits the binding of the spike proteins of the virus to the ACE2 receptor on human cells. Subsequently, entering of the virus into the cells is blocked and therefore also infection of the cells.

As there is no other easy way to directly observe molecules, chemical simulation experiments were used. These - together with the measurements performed - persuasively show that the inhibitory substance Hesperidin stays in the alginate network and can diffuse therein and therefore binds from this reservoir to the spike protein of viral particles and inhibits their binding to the ACE2 receptor.

In this way and according to the present invention, a new and superior combination of at least two antiviral mechanisms is provided. Additionally, a sophisticated mechanism is used in the present invention to provide and use a substance, i.e. calcium alginate, in the prophylaxis of an infection, preferably with SARS-CoV-2, that cannot be sprayed directly, by making use of a present element in a human body fluid.

## Claims

1. A composition comprising hesperidin, citral and a film forming substance, wherein the film forming substance is sodium alginate.

2. Composition according to claim 1, wherein the composition has a viscosity in the range of 5-100 mPa·s, preferably in the range of 10 to 70 mPa·s, more preferably in the range of 20 to 50 mPa·s.

3. Composition according to claim 1 or claim 2, wherein the composition comprises the alginate within a concentration range of 0.2 to 5%, preferably of 1 to 3%, more preferably of 0.5 to 2%, in each case based on the total weight of the composition.

4. Composition according to at least one of the preceding claims, wherein the composition comprises Hesperidin with a concentration of between 0.5 and 100 $\mu$M, preferably between 1 and 50 $\mu$M, more preferably between 5 and 10 $\mu$M.

5. Composition according to at least one of the preceding claims, wherein the composition comprises at least one further excipient selected from the group comprising buffers, alcohols, preservatives, stabilizers and matrix compounds.

6. Composition according to at least one of the preceding claims, wherein the composition comprises at least one further substance selected from the group consisting of eucalyptol, limonene, thymol and carvacrol.

7. Composition according to at least one of the preceding claims for use in the prophylaxis of an infection with an infectious particle, wherein the infectious particle is a SARS-CoV-2 virus.

8. Composition for use according to claim 7, wherein the prophylaxis comprises the prevention of the transmission of SARS-CoV-2 viruses between two or more individuals or comprises the prevention of the transmission of SARS-Cov-2 viruses via infected air or surface.

9. A device, preferably a medical device, comprising a composition according to at least one of claims 1 to 6.

10. Device according to claim 9, wherein the device is a mouth spray, a nasal spray, a bottle, a throat spray and/or includes nose drops, inhalants or a sprayable liquid composition.

11. A delivery system including the composition according to at least one of the claims 1 to 6.

12. Delivery system according to claim 11, wherein the delivery system is an air purification system and/or an air conditioning system or an ultrasonic nebulizer, wherein preferably the air purification system and/or the air conditioning or the ultrasonic nebulizer system comprises at least one solid carrier, preferably a porous solid, a mineral, or a porous polymeric material, that comprises the composition and wherein the system optionally comprises a permeable membrane, or a porous polymeric material and/or optionally comprises a programmable electronic dispenser.

13. A system comprising a device according to at least one of claims 9 or 10 and an air delivery system for delivering a composition including one or more protective, viral inhibiting substances, preferably at least Hesperidin.

**Patentansprüche**

1. Zusammensetzung, umfassend Hesperidin, Citral und einen Filmbildner, wobei es sich bei dem Filmbildner um Natriumalginat handelt.

2. Zusammensetzung nach Anspruch 1, wobei die Zusammensetzung eine Viskosität im Bereich von 5-100 mPa·s, bevorzugt im Bereich von 10 bis 70 mPa·s, bevorzugter im Bereich von 20 bis 50 mPa·s aufweist.

3. Zusammensetzung nach Anspruch 1 oder Anspruch 2, wobei die Zusammensetzung das Alginat in einem Konzentrationsbereich von 0,2 bis 5 %, bevorzugt von 1 bis 3 %, bevorzugter von 0,5 bis 2 %, jeweils bezogen auf das Gesamtgewicht der Zusammensetzung, umfasst.

4. Zusammensetzung nach mindestens einem der vorhergehenden Ansprüche, wobei die Zusammensetzung Hesperidin mit einer Konzentration zwischen 0,5 und 100 μM, bevorzugt zwischen 1 und 50 μM, bevorzugter zwischen 5 und 10 μM umfasst.

5. Zusammensetzung nach mindestens einem der vorhergehenden Ansprüche, wobei die Zusammensetzung mindestens einen weiteren Exzipienten umfasst, der aus der Gruppe, die Puffer, Alkohole, Konservierungsmittel, Stabilisatoren und Matrixverbindungen umfasst, ausgewählt ist.

6. Zusammensetzung nach mindestens einem der vorhergehenden Ansprüche, wobei die Zusammensetzung mindestens eine weitere Substanz umfasst, die aus der Gruppe bestehend aus Eucalyptol, Limonen, Thymol und Carvacrol ausgewählt ist.

7. Zusammensetzung nach mindestens einem der vorhergehenden Ansprüche zur Verwendung bei der Prophylaxe einer Infektion mit einem infektiösen Partikel, wobei es sich bei dem infektiösen Partikel um ein SARS-CoV-2-Virus handelt.

8. Zusammensetzung zur Verwendung nach Anspruch 7, wobei die Prophylaxe die Verhinderung der Übertragung von SARS-CoV-2-Viren zwischen zwei oder mehr Individuen oder die Verhinderung der Übertragung von SARS-CoV-2-Viren über infizierte Luft oder Oberfläche umfasst.

9. Vorrichtung, vorzugsweise medizinische Vorrichtung, umfassend eine Zusammensetzung nach mindestens einem der Ansprüche 1 bis 6.

10. Vorrichtung nach Anspruch 9, wobei die Vorrichtung ein Mundspray, ein Nasenspray, eine Flasche, ein Rachenspray ist und/oder Nasentropfen, Inhalationsmittel oder eine sprühbare flüssige Zusammensetzung umfasst.

11. Zuführungssystem, enthaltend die Zusammensetzung nach mindestens einem der Ansprüche 1 bis 6.

12. Zuführungssystem nach Anspruch 11, wobei es sich bei dem Zuführungssystem um ein Luftreinigungssystem und/oder ein Klimatisierungssystem oder einen Ultraschallvernebler handelt, wobei vorzugsweise das Luftreinigungssystem und/oder das Klimatisierungs- oder das Ultraschallverneblersystem mindestens einen festen Träger, bevorzugt einen porösen Feststoff, ein Mineral oder ein poröses Polymermaterial, umfasst, der die Zusammensetzung umfasst, und wobei das System gegebenenfalls eine permeable Membran oder ein poröses Polymermaterial umfasst und/oder gegebenenfalls einen programmierbaren elektronischen Spender umfasst.

13. System, umfassend eine Vorrichtung nach mindestens einem der Ansprüche 9 oder 10 und ein Luftzuführungssystem zur Zuführung einer Zusammensetzung, die einen oder mehrere schützende, virenhemmende Substanzen, bevorzugt mindestens Hesperidin, enthält.

## Revendications

1. Composition comprenant de l'hespéridine, du citral et une substance filmogène, la substance filmogène étant l'alginate de sodium.

2. Composition selon la revendication 1, la composition ayant une viscosité comprise entre 5 et 100 mPa·s, de préférence entre 10 et 70 mPa·s, plus préférentiellement entre 20 et 50 mPa·s.

3. Composition selon la revendication 1 ou la revendication 2, la composition comprenant l'alginate à une concentration comprise entre 0,2 et 5 %, de préférence entre 1 et 3 %, plus préférentiellement entre 0,5 et 2 %, dans chaque cas par rapport au poids total de la composition.

4. Composition selon au moins l'une des revendications précédentes, la composition comprenant de l'hespéridine à une concentration comprise entre 0,5 et 100 $\mu$M, de préférence entre 1 et 50 $\mu$M, plus préférentiellement entre 5 et 10 $\mu$M.

5. Composition selon au moins l'une des revendications précédentes, la composition comprenant au moins un excipient supplémentaire choisi dans le groupe comprenant des tampons, des alcools, des conservateurs, des stabilisants et des composés matriciels.

6. Composition selon au moins l'une des revendications précédentes, la composition comprenant au moins une substance supplémentaire choisie dans le groupe constitué par l'eucalyptol, le limonène, le thymol et le carvacrol.

7. Composition selon au moins l'une des revendications précédentes, destinée à être utilisée dans la prophylaxie d'une infection par une particule infectieuse, la particule infectieuse étant un virus SARS-CoV-2.

8. Composition selon la revendication 7, la prophylaxie comprenant la prévention de la transmission de virus SARS-CoV-2 entre au moins deux individus, ou la prévention de la transmission de virus SARS-CoV-2 par l'intermédiaire de l'air infecté ou d'une surface infectée.

9. Dispositif, de préférence un dispositif médical, comprenant une composition selon au moins l'une des revendications 1 à 6.

10. Dispositif selon la revendication 9, le dispositif étant un spray buccal, un spray nasal, un flacon, un spray pour la gorge et/ou comprend des gouttes nasales, des formes pour inhalation ou une composition liquide pulvérisable.

11. Système d'administration comprenant la composition selon au moins l'une des revendications 1 à 6.

12. Système d'administration selon la revendication 11, ledit système d'administration étant un système de purification d'air et/ou un système de climatisation ou un nébuliseur ultrasonique, de préférence, le système de purification d'air et/ou le système de climatisation ou le nébuliseur ultrasonique comprenant au moins un support solide, de préférence un solide poreux, un minéral ou un matériau polymère poreux, qui contient la composition, et le système comprenant facultativement une membrane perméable, ou un matériau polymère poreux et/ou comprenant facultativement un distributeur électronique programmable.

13. Système comprenant un dispositif selon au moins l'une des revendications 9 ou 10 et un système d'administration entraîné par l'air pour administrer une composition comprenant une ou plusieurs substances protectrices, inhibitrices de virus, de préférence au moins l'hespéridine.

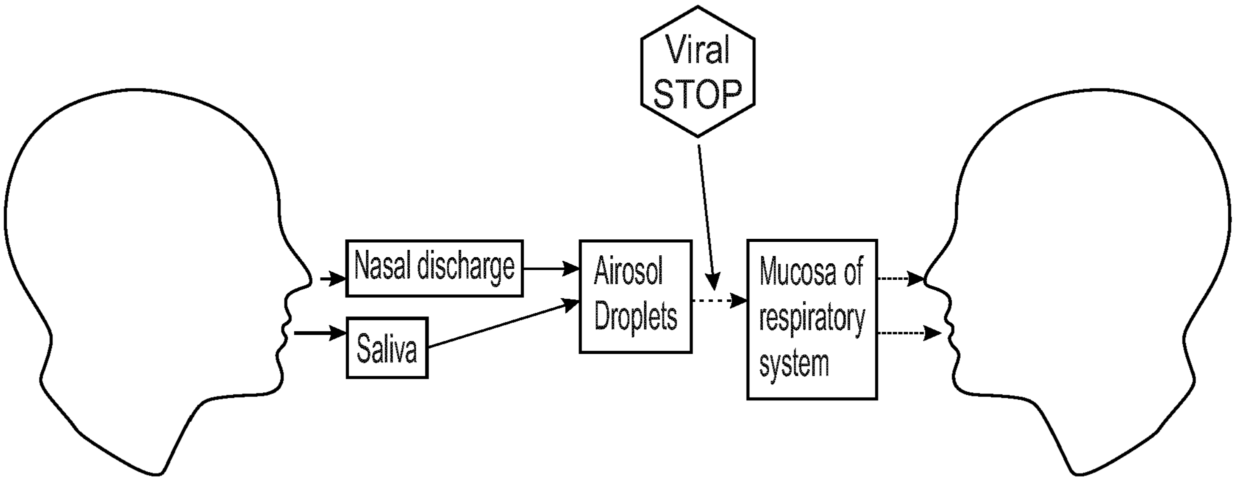
Viral
STOP
Nasal discharge
Saliva
Airosol
Droplets
Mucosa of
respiratory
system

Fig. 1

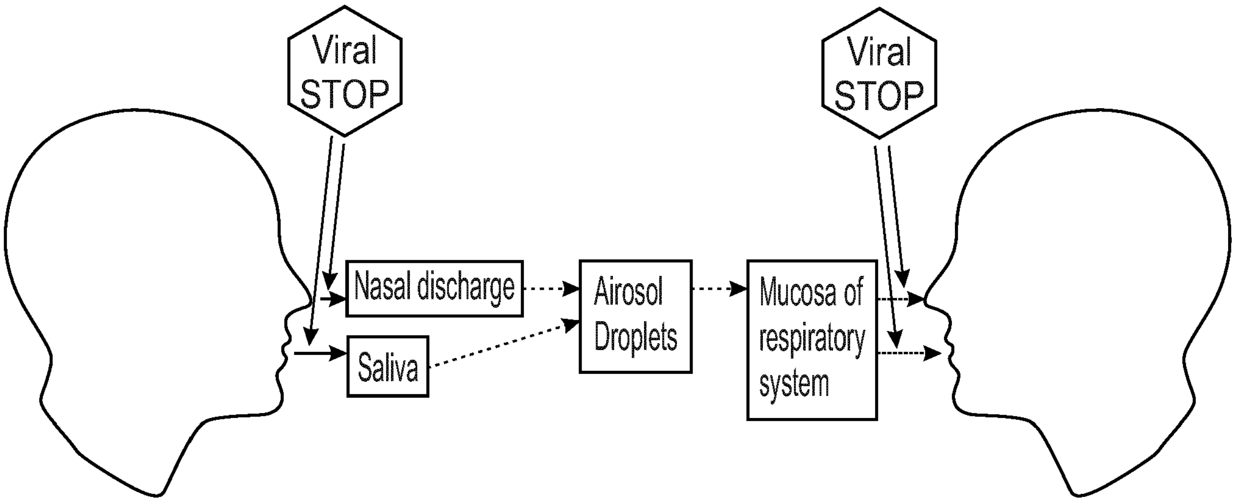
Viral
STOP
Viral
STOP
Nasal discharge
Saliva
Airosol
Droplets
Mucosa of
respiratory
system

Fig. 2

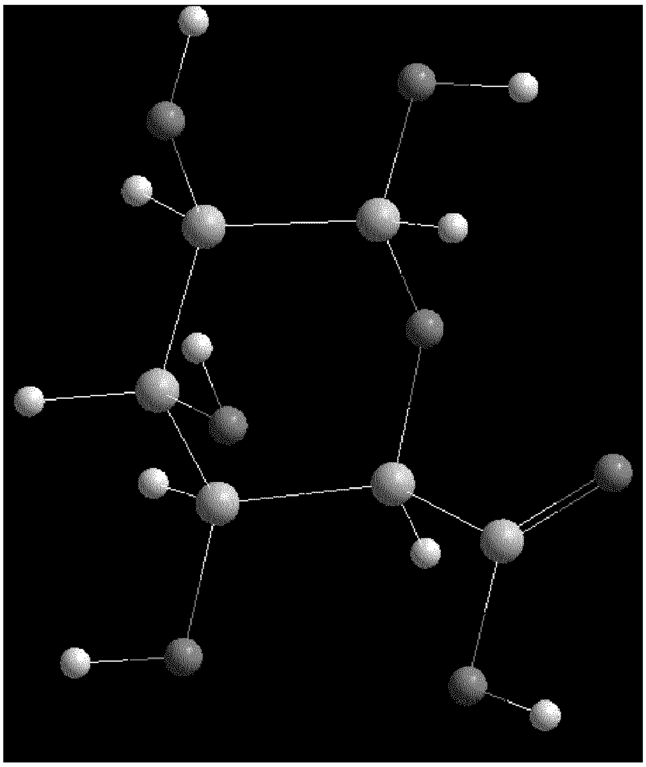

Fig. 3

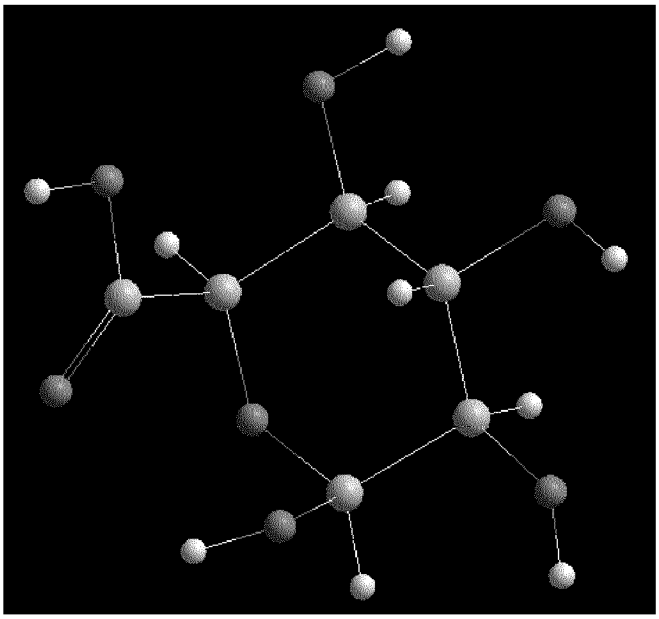

Fig. 4

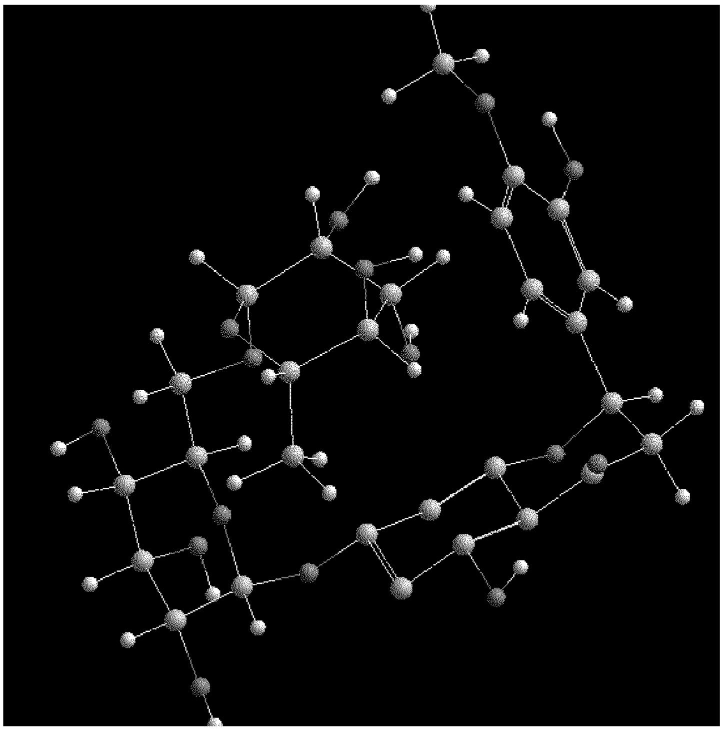

Fig. 5

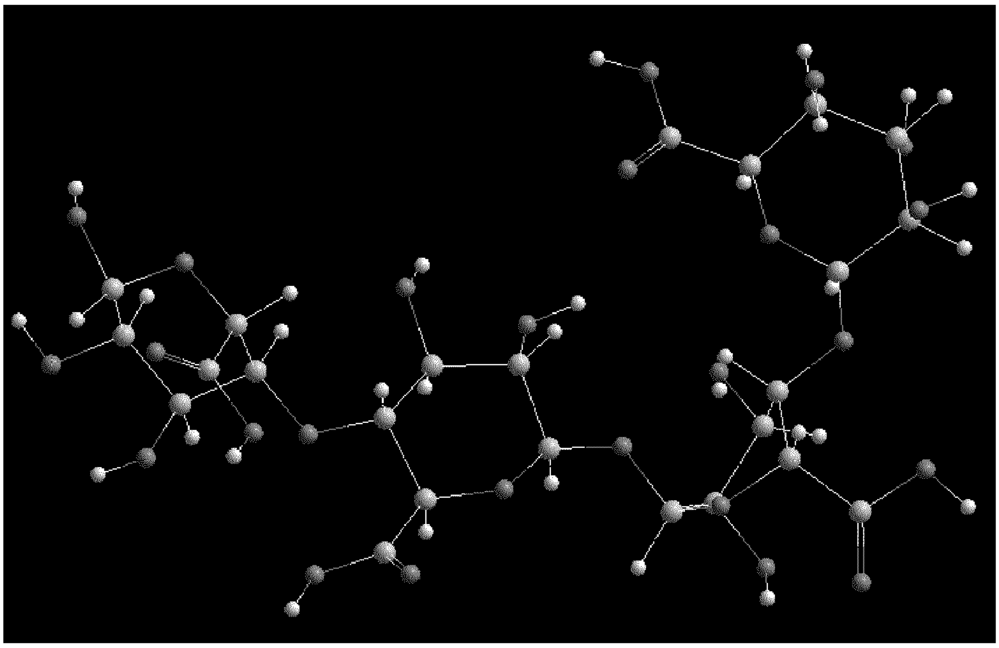

Fig. 6

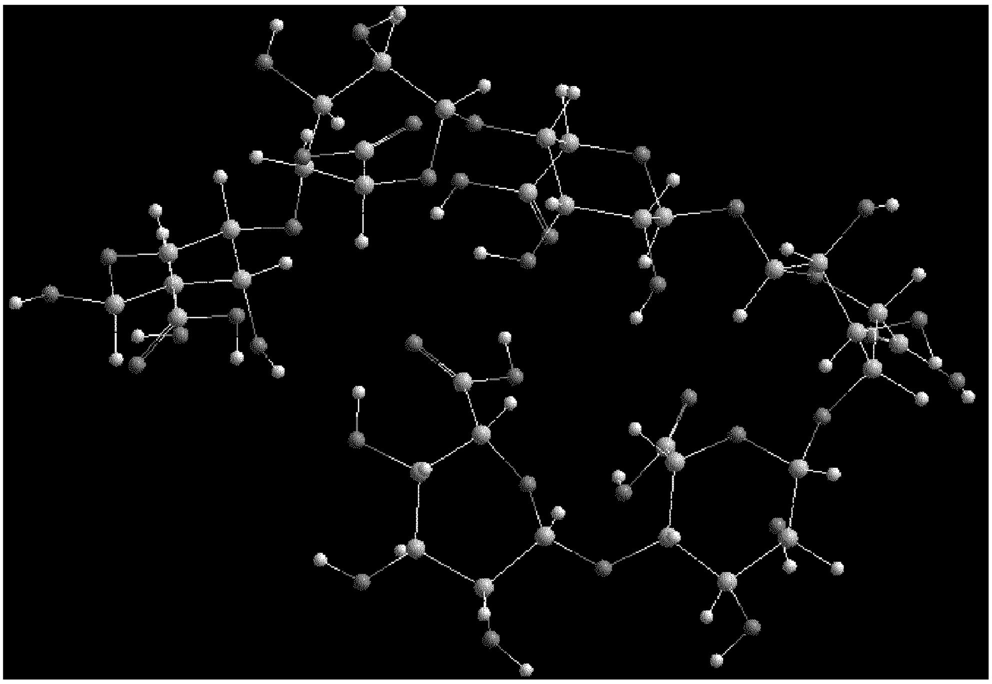

Fig. 7

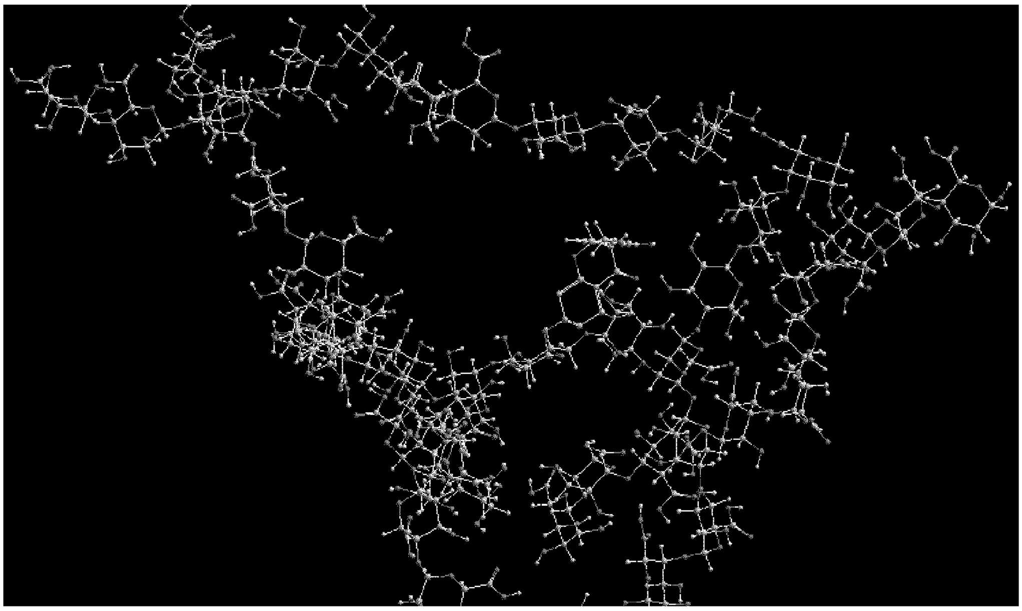

Fig. 8

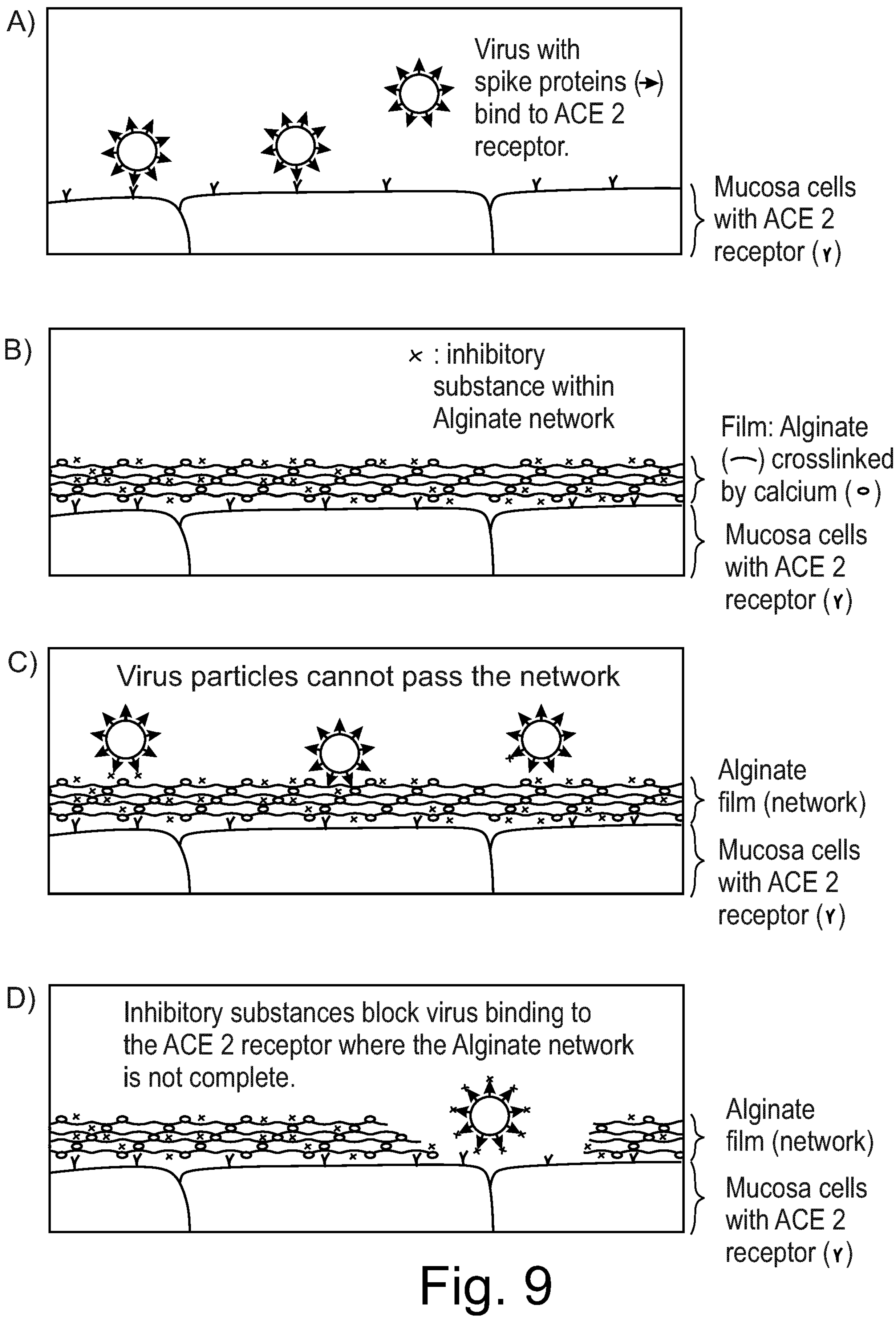
A)
Virus with
spike proteins (→)
bind to ACE 2
receptor.
Mucosa cells
with ACE 2
receptor (Y)
B)
× : inhibitory
substance within
Alginate network
Film: Alginate
(⁀) crosslinked
by calcium (∘)
Mucosa cells
with ACE 2
receptor (Y)
C)
Virus particles cannot pass the network
Alginate
film (network)
Mucosa cells
with ACE 2
receptor (Y)
D)
Inhibitory substances block virus binding to
the ACE 2 receptor where the Alginate network
is not complete.
Alginate
film (network)
Mucosa cells
with ACE 2
receptor (Y)

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MENEGUZZO et al.** Review of Evidence Available on Hesperidin-Rich Products as Potential Tools against COVID-19 and Hydrodynamic Cavitation-Based Extraction as a Method of Increasing Their Production. *PROCESSES*, 09 April 2020, vol. 8 (5), 549
- **HAGGAG et al.** Is hesperidin essential for prophylaxis and treatment of COVID-19 Infection?. *Medical Hypotheses*, November 2020, vol. 144, 109957
- **O. TROTT** ; **A. J. OLSON**. AutoDock Vina: improving the speed and accuracy of docking with a new scoring function, efficient optimization and multithreading. *Journal of Computational Chemistry*, 2010, vol. 31, 455-461